# EUROPEAN PATENT APPLICATION

(11) **EP 1 992 347 A1**
(43) Date of publication of application: **19.11.2008**
(21) Application number: 07009941.1
(22) Date of filing: 18.05.2007
(51) Int. Cl.: A61K 31/506, A61P 35/00, A61N 5/00

(54) **Treatment of DDR1 positive cancer using imatinib**

(71) Applicant: CELLZOME AG, 69117 Heidelberg (DE)
(72) Inventor: The designation of the inventor has not yet been filed
(74) Representative: Huhn, Michael

(57) **Abstract**

The present invention relates to the methods for the treatment of DDR1 positive cancer using Imatinib.

## Description

The present invention relates to methods for the the treatment of Discoidin Domain Receptor 1 (DDR1) positive cancers using Imatinib.

Kinases catalyze the phosphorylation of proteins, lipids, sugars, nucleosides and other cellular metabolites and play key roles in all aspects of eukryotic cell physiology. Especially, protein kinases and lipid kineses participate in the signaling events which control the activation, growth, differentiation and survival of cells in response to extracellular mediators or stimuli such as growth factors, cytokines, chemokines or antigens. In general, protein kinases are classified in two groups, those that preferentially phosphorylate tyrosine residues and those that preferentially phosphorylate serine and/or threonine residues.

Inappropriately high protein kinase activity is involved in many diseases including cancer (e.g. solid tumours and hematological disorders) and autoimmune/inflammatory disorders. This can be caused either directly or indirectly by the failure of control mechanisms due to mutation, overexpression or inappropriate activation of the enzyme. In all of these instances, selective inhibition of the kinase is expected to have a beneficial effect.

Currently the pharmaceutical industry devotes substancial efforts to develop disease-specific, molecularly targeted therapeutics directed at individual kinases or a small subset of kinases involved in disease initiation or progression. In addition, the identification and selection of patient subpopulations that respond to a particular drug becomes increasingly important for the design of effective treatment regimens.

The prototype of a targeted kinase inhibitor is Imatinib (also known as STI-571, Gleevec®, or Glivec®) which inhibits a subset of kinases comprising the fusion protein Bcr-Abl, c-Abl, PDFG receptors and the c-Kit receptor (Capdeville et al., 2002. Nature Reviews Drug Discovery 1, 493-502). Imatinib is a phenylaminopyrimidine derivative (4-(4-methylpiperazin-1-ylmethyl)-N-[4-methyl-3-(4-pyridin-3-yl)pyrimidin-2-ylamino)phenylbenzamide).

Imatinib is efficacious against both chronic myelogenous leukemia (CML) and gastrointestinal stromal tumors (GIST) (Manley et al., 2002, European Journal of Cancer 38 Suppl. 5, S19-S27; Buchdunger et al., 2002, European Journal of Cancer 38, Suppl. 5, S28-S36). A subset of gastrointestinal stromal tumors (GIST) harbours mutations of the c-Kit gene and permanently expresses acivated forms of the c-Kit receptor. Interestingly, about 30 % of GISTs lacking mutations in c-Kit have mutations in the platelet-derived growth factor receptor alpha (PDGFRA) gene, resulting in constitutively active PDGFRalpha. In GIST the therapeutic effect of Gleevec is presumably mediated by c-Kit and/or PDGF receptors and Imatinib could be viewed as multi-targeted inhibitor. In both diseases the identification of patients that are expected to respond to Gleevec treatment is an important step, Bcr-Abl positive patients for CML and mutated c-Kit or PDGFRA in GIST (Baselga, 2006, Science 312, 1175-1178; Sebolt-Leopold and English, 2006, Nature 441, 457-462).

Discoidin Domain Receptors (DDR1) proteins are receptor tyrosine kinases that function as receptors for collagen. To date, two family members, DDR1 and DDR2 are known. After collagen binding DDRs regulate cell adhesion, proliferation and remodelling of the extracellular matrix (Vogel et al., 2006. Cellular Signalling 18, 1108-1116). The structure of DDRs differs from other receptor tyrosine kinases by the presence of a discoidin domain, an approximately 16 amino acid residue long holomogy region first identified in the the protein discoidin frome the slime mold Dictyostelium discoideum.

The genomic structure of the DDR1 gene was anaylzed and found to comprise 15 exons (Sakuma et al., 1996, FEBS Letters 398, 165-169). To date, five isoforms of DDR1 have been identified which are generated by alternative splicing in the cytoplasmic region (Alves et al., 2001, The FASEB Journal 15, 1321-1323). In several cell lines and tissues DDR1 is partially processed into a 62 kDa menbrane-anchored beta-subunit and a 54 kDa soluble extracellular domain-containing aplpha subunit (Vogel, 2002. FEBS Letters 514, 175-180).

DDRs have been linked to a number of diverse human cancers by studies of tumor cell lines and clinical samples (summarized in Vogel et al., 2006. Cellular Signalling 18, 1108-1116).

DDR1 was suggested as a marker to distinguish lobular and ductal invasive breast carcinomas (Turashvili et al., 2007. BMC Cancer 7, 55). Another study suggested that DDR1a promotes glioma cell invasion (Ram et a., 2006. Journal of Neurooncology 76, 239-248). Overexpression of DDR1 was reported for metaplastic ovarian epithelium and ovarian cancer (Heinzelmann-Schwarz et al., 2004. Clinical Cancer Research 10, 4427-4436). In another study it was reported that DDR1 enhances cell invasion and adhesion in pituitary adenoma (Yoshida and Teramoto, 2007. J. Neurooncol. 82, 29-40). Furthermore, it was demonstrated that DDR1 contributes to eosinophil survival in a NF-kappaB-dependent manner in Churg-Strauss syndrome (Matsuyama et al., 2007. Blood 109, 22-30).

In addition, it was shown that DDR1 is a direct transcriptional target of the p53 tumor suppressor gene and that DNA damage induced a p53-dependent DDR1 response associated with activation of its kinase function (Ongussaha et al, 2003. EMBO Journal 22, 1289-1301). Dominant-negative DDR1 inhibited irradiation-induced MAPK activation. The inhibition of DDR1 function resulted in increased apoptosis of cells containing wild-type p53 in response to genotoxic stress.

Several reports described that treatment of cells with Imatinib enhances their sentivity towards radiation. For example, it was shown that Imatinib radiosensitizes human glioblastoma cells and it was suggested that this effect is meadiated by inhibition of the PDGF receptor (Holdhoff et al., 2005. Blood Cells, Molecules & Diseases 34, 181-185). Another study investigated the combination of Imatinib, retinoic acid and γ-radiation treatment of neuroblastoma cell lines. The cell viability was inhibited at various concentration of Imatinib. However, the cell lines responded to Imatinib irrespective of the expression of the known Imatinib targets c-Kit, PDGFR-α and PDGFR-β (Rössler et al., 2006, Biochem. Biophys. Res. Comm. 342, 1405-1412).

The present invention is directed to the use of Imatinib for the preparation of a medicament for treating a patient having a DDR1 positive cancer.

In the context of the present invention, it has been surprisingly found that Imatinib potently binds to the Discoidin Domain Receptor 1 (DDR1) and inhibits DDR1 kinase activity, thus suggesting the use of Imatinib for the treatment of DDR1 dependent diseases.

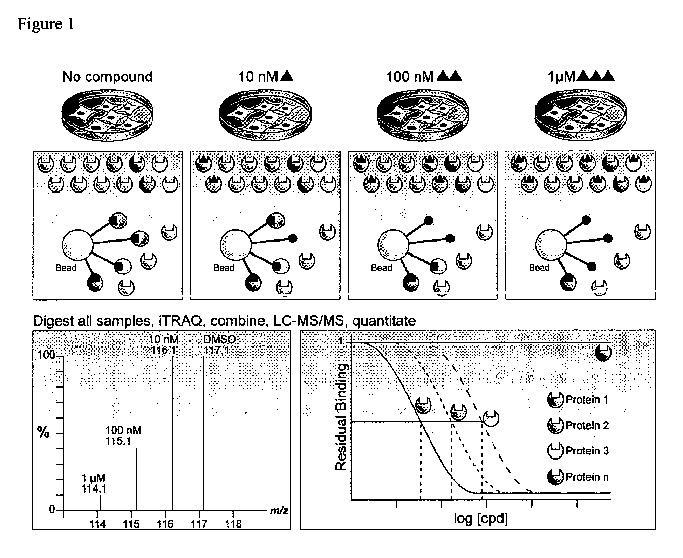

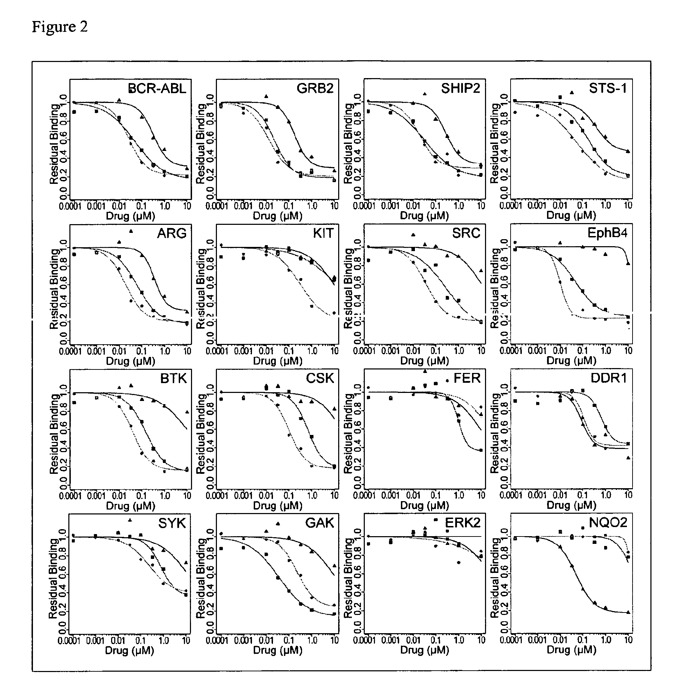

In a competitive binding experiment Imatinib was incubated with a cell lysate containing DDR1 and free DDR1 was captured by an affinity matrix consisting of immobilized capture compounds (kinobeads; Example 1; Figure 1). This experiment revealed that Imatinib tightly interacts with DDR1 as shown by dose-response curves (Figure 2).

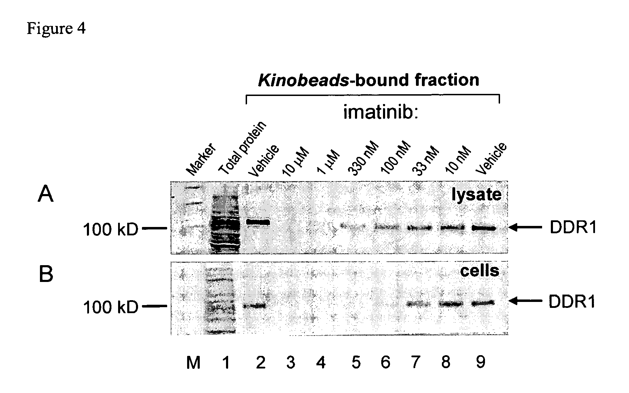

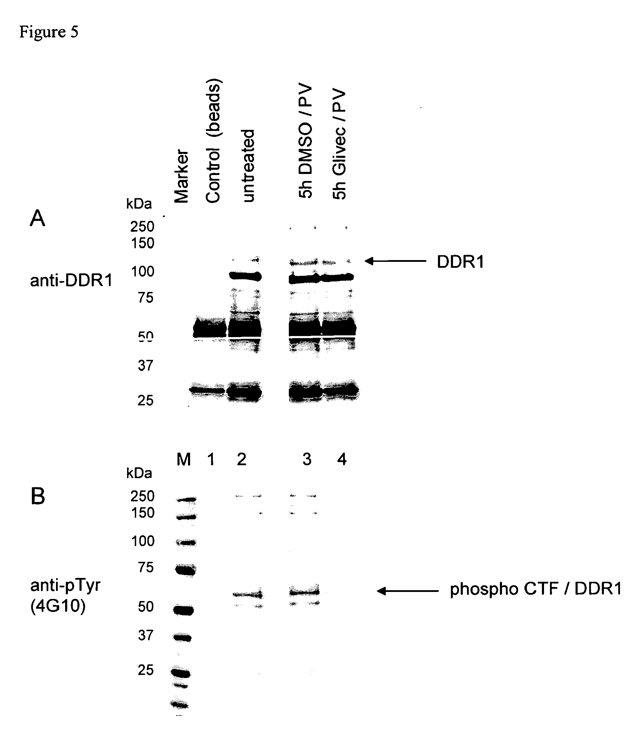

In a separate experiment Imatinib was added to cells and subsequently cell lysates were generated and subjected to kinobeads analysis also revealing a tight interaction with DRR1. In addition, samples from both experiments were analysed by Western blot analysis (Figure 4). Furthermore it was shown that Imatinib not only interacts with DDR1 but also inhibits DDR1 kinase activity (Figure 5).

As already discussed above, the Imatinib drug used in the context of the present invention is known in the art (Buchdunger et al., 2002, European Journal of Cancer 38, Suppl. 5, S28-S36). Imatinib is commercially available. Furthermore, since Imatinib is an established drug, its administration is also known in the art.

In the context of the present invention "DDR1" means Discoidin Domain Receptor 1 (reviewed in Vogel et al., Cellular Signalling 18, 1108-116). The genomic structure of the DDR1 gene was anaylzed and found to comprise 15 exons (Sakuma et al., 1996, FEBS Letters 398, 165-169). To date five isoforms of DDR1 have been identified which are generated by alternative splicing in the cytoplasmic region (Alves et al., 2001, The FASEB Journal 15, 1321-1323). In several cell lines and tissues DDR1 is partially processed into a 62 kDa menbrane-anchored beta-subunit and a 54 kDa soluble extracellular domain-containing aplpha subunit (Vogel, 2002. FEBS Letters 514, 175-180).

In the context of the present invention, the term "cancer" comprises solid neoplastic tumours (for example brain, breast, lung and colon cancer) and leukemias (for example chronic lymphocytic leukemia and chronic myelogenous leukemia).

Cancer comprises a group of diseases characterized by uncontrolled growth and spread of abnormal cells. All types of cancers generally involve some abnormality in the control of cell growth, division and survival, resulting in the malignant growth of cells. Key factors contributing to said malignant growth of cells are independence from growth signals, insensitivity to anti-growth signals, evasion of apoptosis, limitless replicative potential, sustained angiogenesis, tissue invasion and metastasis, and genome instability (Hanahan and Weinberg, 2000. The Hallmarks of Cancer. Cell 100, 57-70).

According to the present invention, a "DDR1 positive cancer" means that at least some cells of said cancer express DDR1. Test for measuring DDR1 expression are known in the (see below).

According to a preferred embodiment, the DDR1 positive cancer is selected from the group consisting of brain cancer, breast cancer, and lung cancer.

According to a further preferred embodiment of the invention, at least one cancer cell of the patient is tested for the expression of DDR1 prior to the administration of Imatinib and the patient is only treated if DDR1 is expressed in said cancer cell. This enables a specific treatment of patients having a DDR1 positive cancer.

Preferably, said testing is performed by measuring the presence of DDR1 protein or by measuring the presence of an mRNA encoding DDR1.

According to the invention, the presence of DDR1 protein may be measured by any method known in the art to measure protein expression, including immunohistochemistry, Western blot or flow cytometry. Specific methods for deceting the expression of DDR1 by immunohistochemistry, also in clinical specimen, are known in the art (Turashvili et al. 2007. BMC Cancer 7, 55).

Alternatively, the expression of DDR1 in cells can be tested by Western blot analysis using an anti-DDR1 antibodiy (Alves et al., 2001, FASEB J. 15, 1321-1323).

Flow cytometry (FC) can quantify DDR1 on the surface of cells, but requires the use of viable cells. The multiparametric analysis of flow cytometry allows a assay using an unpurified sample of cells (Matsuyama et al., 2006, Am. J. Pathol. 168, 866-877).

According to the invention, the presence of the mRNA may be measured by any method known to the skilled person including Northern Blot, in situ hybridization, quantitative RT-PCR or DNA microarrays. Specific methods to measure the expression level of DDR1 encoding mRNA by quantitative real-time PCR are known in the art (Ford et al., British Journal of Cancer 96, 808-814). In addition, detection methods for DDR1 mRNA using DNA microarrays have been published (Turashvili et al., 2007. BMC Cancer 7, 55).

In a preferred embodiment of the invention, Imatinib is administered in combination with radiation therapy. This is especially useful since it is known in the art that Imatinib may increase the sensitivity for radiation therapy. Current treatments with radiation therapy for cancer are known in the art.

Radiotherapy may be administered according to the known practices in clinical radiotherapy. The necessary dosages of ionising radiation are known in the art. Radiation therapy includes the use of γ-rays, X-rays, and/or the directed delivery of radiation from radioisotopes. For example,X-rays may be dosed in daily doses of 1.8 to 2.0 Gy, 5 days a week for 5 to 6 weeks. Normally a total fractinated dos will lie in the range of 45 to 60 gy.

Single larger doses, for example 5 to 10 Gy, may be administered during the course of raiotherapy. Doses ranges for radioisotopes vary widelyand depend on the half-life of the isotope, the type and strength of the emitted radiation, and on the uptake by cells.

In this context, Imatinib may be admistered before the onset of the radiation therapy. However, it is also envisaged in the context of the present invention that Imanitinib is used during radiation therapy, especially when it is envisaged to use multiple therapy cycles.

The present invention further relates to a method for treating a patient having a DDR1 positive cancer, comprising administering to the patient a pharmacological effective amount of Imatinib.

All preferred embodiments and definitions discussed above with respect to the use of the invention also apply to the this method of the invention.

Especially, the DDR1 positive cancer is selected from the group consisting of brain cancer, breast cancer, and lung cancer.

Preferably, at least one cancer cell of the patient is tested for the expression of DDR1 prior to the administration of the Imatinib and the patient is only treated if DDR1 is expressed in said cancer cell.

Preferably, said testing is performed by measuring the protein level of DDR1 or by measuring the presence of an mRNA encoding DDR1.

According to a further preferred embodiment, Imatinib is administered in combination with radiation therapy.

The invention will be further described with the help of the following examples and figures, which are intended to illustrate but not to limit the scope of the present invention.

### Short description of the figures

**Figure 1****:** Schematic overview of the kinobeads assay.
   Either lysates or cells are treated with increasing concentrations of inhibitor or without inhibitor (no compound, DMSO control). Subsequently, proteins are captured on kinobeads. The inhibitor competes with the immobilized ligands for ATP- or other purine-binding sites of its targets. Bound proteins are digested with trypsin and each peptide pool is labeled with iTRAQ reagent. All four samples are combined and analyzed by mass spectrometry. Each peptide gives rise to four characteristic iTRAQ reporter signals indicative of the inhibitor concentration used. For each peptide detected, the decrease of signal intensity compared to the vehicle control reflects competition by the 'free' drug for its target.
**Figure 2****:** Competition binding curves calculated from iTRAQ reporter signals.
   Binding of selected known and novel targets to kinobeads is shown as dependent on the addition of imatinib (triangles, blue), dasatinib (cicrles, green), and bosutinib (squares, red) to K562 cell lysate.
**Figure 3****:** Kinase binding profiles of the Abl inhibitors imatinib, dasatinib and bosutinib across a set of protein kinases simultaneously identified from K562 cells.
   The bars indicate the IC₅₀ values defined as the concentration of drug at which half-maximal competition of kinobeads binding is observed.
**Figure 4****:** Western blot analysis of proteins captured on kinobeads detects DDR1 as a target of Imatinib.
   A: Imatinib treatment of K562 cell lysate reduces the amount of DDR1 captured on kinobeads.
   B: Imatinib treatment of K562 cells in culture similarly reduces the amount of DDR1 captured on kinobeads.
**Figure 5****:** Inhibition of DDR1 autophosphorylation by imatinib.
   K562 cells were treated with 30 µM pervanadate for 30 minutes to induce tyrosine autophosphorylation of DDR1. DDR1 was analyzed by immunoprecipitation with bead-coupled anti-DDR1 antibodies (Santa cruz sc-532 AC) and Western blotting with anti-DDR1 and anti-phosphotyrosine antibodies. In lane 2 the immunobeads were analysed revealing that the two strong beads result from the anti-DDR1 antibodies coupled to the beads. Pre-incubation (3 hours) of the cells with 1 µM imatinib reduces the pervanadate-induced tyrosine phosphorylation and phosphorylation-mediated degradation of DDR1 (lane 4).
   A: Western blot probed with anti-DDR1 antibody (Santa Cruz sc-532).
   B: Western blot probed with anti-phosphotyrosine (anti-pTyr) antibody (Upstate catalogue number 05-321, clone 4G10).

### Example 1: Quantitative target profiling of drugs in cell lysates

We applied the kinobeads methods (WO 2006/134056A1) to the quantitative profiling of three inhibitors of the tyrosine kinase ABL; the drug candidate bosutinib (SKI-606) (Boschelli et al., 2004, J. Med. Chem. 47, 1599-1601) which is currently in clinical studies and the marketed drugs imatinib (Capdeville et al., 2002. Nat. Rev. Drug Discovery 1, 493-502) and dasatinib (Das et al., 2006, J. Med. Chem. 49, 6819-6832). All experiments were performed using K562 chronic myeloid leukemia cells, which express the constitutively active BCR-ABL oncogene (Druker et al., 1996, Nat. Med. 2, 561-566. The drugs were added to K562 lysates in concentrations ranging from 100 pM to 10 µM and the lysates were subsequently subjected to kinobeads precipitation. When the drug in the lysate binds its target and thus blocks the ATP binding site, a reduced amount of the free target is available for capturing on kinobeads, while the binding of non-targeted kinases and other proteins is unaffected (Figure 1). The kinobeads-bound material from each spiking experiment was subjected to tryptic digestion and peptides were labeled with the different forms of the iTRAQ reagent (Ross et al., 2004, Mol. Cell. Proteomics 3, 1154-1169). Subsequently, peptide mixtures were combined and subjected to mass spectrometric peptide sequencing. Relative protein quantification was achieved by measuring the signal of the iTRAQ reporter ions relative to vehicle-treated lysate. From this dataset, dose-response binding profiles were computed.

For imatinib, 13 proteins exhibited more than 50% binding reduction on kinobeads at 1 µM drug in the lysate. Among the competed proteins are ABL/BCR-ABL (IC50 = 250 nM), the ABL family kinase Arg (272 nM), and two novel target candidates, the receptor tyrosine kinase DDR1 (90 nM), and the quinone oxidoreductase NQO2 (43 nM) (Figure 2).

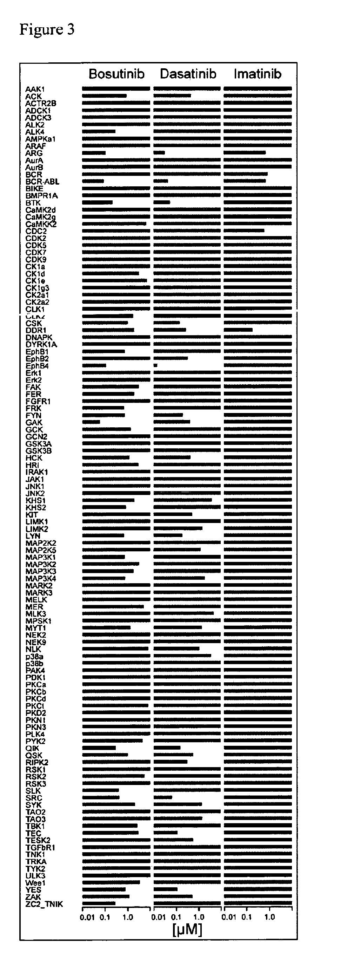

While imatinib affected only three out of the more than 140 kinases that were quantified in K562 lysate, dasatinib and bosutinib reveal broad target profiles (46 and 42 proteins respectively showed >50% competition at 1 µM), including the three imatinib targets ABL/BCR-ABL, ARG, and DDR1 (Figure 3). The majority of the novel kinase targets were not available in commercial kinase panels, but for the tyrosine kinases Btk, EphB4, FAK/PTK2, FER, MER, and SYK, and the serine/threonine kinases GCK, KHS1 and p38a we determined IC50 values in enzyme activity assays, which show a general trend supporting the kinobeads data.

A notable exception is the focal adhesion kinase FAK/PTK2, which was affected only by bosutinib, in line with published data reporting no binding of dasatinib or imatinib to FAK in phage display studies (Fabian et al., 2005, Nat. Biotechnol. 23, 329-336). However, a diphosphorylated peptide representing the activation segment of the FAK kinase domain was strongly affected by dasatinib, suggesting that the drug binds selectively to the activated conformation. In agreement with this interpretation, dasatinib potently inhibited the activity of purified recombinant FAK.

In addition to kinases, several non-kinase targets were identified, some of which do not contain obvious small molecule binding sites and hence are likely to bind indirectly to the drugs. Proteins which reside in a complex with the drug target are expected to exhibit similar competition behaviour. Indeed the BCR-ABL interacting proteins GRB2, SHC1, and SHIP2 displayed similar competition behaviour. STS-1, an adaptor protein described to inhibit the ubiquitin ligase CBL (Kowanetz et al., 2004, J. Biol. Chem. 279, 32786-32795) , also showed a similar dose-response for all three drugs. Consequently we propose it as a BCR-ABL/ABL kinase interacting protein (Figure 2).

In addition to the ABL kinases, imatinib is also known to inhibit oncogenic mutants of the KIT and PDGF receptors, which is the basis of its therapeutic application in gastrointestinal stromal tumours (Tuveson et al., 2001, Oncogene 20, 5054-5058). PDGF receptors are not expressed in K562 cells. Although K562 cells do express wild type KIT, no significant competition of imatinib for kinobeads-captured KIT was detected by mass spectrometry. Likewise bosutinib did not significantly affect KIT, but dasatinib showed potent binding (IC₅₀ = 0.30 µM). This observation was confirmed by Western blot analysis of the kinobeads-captured material from imatinib-treated lysates using KIT antibodies. However, when the same blots were probed for activated KIT using a phospho-specific antibody directed against tyrosine 703, sub-micromolar competition by imatinib was seen. Hence, the kinobeads binding assay can differentiate between binding of a drug to distinct conformations of a target present in the same cell.

### Novel targets of imatinib

The discoidin domain receptor kinase DDR1 represents a novel imatinib target. We confirmed the dose-response established by mass spectrometry by probing the same samples with DDR1 antibodies (Figure 4). Next, we tested whether imatinib inhibits the DDR1 kinase activity. DDR1 is a receptor tyrosine kinase which exhibits autophosphorylation and limited proteolysis in response to collagen binding or pervanadate treatment (L'hote et al., 2002, FASEB J. 16, 234-236). Pre-incubation of the cells with imatinib reduced tyrosine phosphorylation and proteolytic processing of DDR1 (Figure 5).

### Profiling of drug effects on signaling pathways

Potent kinase inhibitors typically exhibit slow off-rates, which permits a variation of the previous experimental strategy. Instead of adding the drugs to the lysate, we applied them over a range of concentrations to cultured cells 5 hours prior to lysis and kinobeads precipitation (WO 2006/134056A1). The results confirmed almost all of the targets obtained with the previous lysate competition experiments.

To explore not only the direct targets of the drugs but also their downstream effects on signaling pathways, aliquots of the iTRAQ-labeled peptide mixtures from kinobeads precipitates were subjected to phosphopeptide enrichment and subsequent identification and quantification by mass spectrometry. For imatinib-treated cells, 351 tyrosine and serine/threonine phosphorylation sites on 119 different proteins were identified. Fourteen of these sites on 9 different proteins exhibited significant down-regulation of their phosphorylation status in response to the drug. Indeed, several of these proteins have been implicated in signaling events downstream of ABL. For dasatinib and bosutinib the data are more complex reflecting their expanded target profile.

**Kinobeads and competition assays.** Reagents were purchased from Sigma unless otherwise noted. Compounds for immobilization to beads were synthesized as described (WO 2006/134056A1). Kinobeads were prepared by immobilizing Bis-(III) Indolyl-maleimide, purvalanol B, staurosporine, and CZC8004, and the analogues of PD173955, sunitinib, and vandetanib on NHS-activated Sepharose 4 beads (Amersham) as described (WO 2006/134056A1). Imatinib was purified from Gleevec tablets (Novartis) by HPLC. Dasatinib and Bosutinib were synthesized following published procedures (Das et al., 2006, J. Med. Chem. 49, 6819-6832; Boschelli et al., 2004, J. Med. Chem. 47, 1599-1601). HeLa and K562 cells were obtained from ATCC and were cultured following ATCC protocols. Antibodies were purchased from Cell Signaling Technology (KIT, Y703P-KIT) and Santa Cruz (DDR1; catalogue number sc-532) and Western blots were performed using a LI-COR Odyssey Imaging System.

Kinobeads profiling was performed as described (WO 2006/134056A1). Briefly, cells were homogenized in lysis buffer (50 mM Tris/HCl pH 7.5, 5% glycerol, 1.5 mM MgCl₂, 150 mM NaCl, 20 mM NaF, 1 mM Na₃VO₄, 1 mM DTT, 5 µM Calyculin A, 0.8 % Igepal-CA630, and a protease inhibitor cocktail) using a Dounce homogenizer on ice. Lysates were and cleared by centrifugation and adjusted to 5 mg/ml protein concentration using the Bradford assay. Compounds were dissolved in DMSO and added to 5 ml lysate samples, and 50 µl of a kinobeads suspension was added and agitated for 30 minutes at 4°C. For profiling of signaling pathways, compound were added to 10⁸ K562 cells per data point, grown at a density of 10⁶ cells/mL in RPMI/10% FCS. Beads were eluted with NuPAGE SDS-containing buffer, eluates were reduced, alkylated, separated on 4-12% NuPAGE gels (Invitrogen), and stained with colloidal Coomassie.

**Mass Spectrometry and Data analysis**. Procedures were performed as described (WO 2006/134056A1). Briefly, gel lanes were cut into slices across the separation range and subjected to in-gel tryptic digestion (Shevchenko et al., 1996, Anal. Chem. 68, 850-858), followed by labeling with iTRAQ^{™} reagents (Applied Biosystems) as described (Ross et al., 2004, Mol. Cell. Proteomics 3, 1154-1169). Labeled peptide samples were combined and phosphopeptides were enriched using immobilized metal affinity chromatography (PhosSelect, Sigma) (Pozuelo et al., 2005, Biochem. J. 392, 163-172). Sequencing was performed by LC-MS/MS on an Eksigent 1D+ HPLC system coupled to a LTQ-Orbitrap mass spectrometer (Thermo Scientific). Peptide extracts of vehicle controls were labeled with iTRAQ reagent 117 and combined with extracts from compound-treated samples labeled with iTRAQ reagents 114-116. Tandem mass spectra were generated using pulsed-Q dissociation, enabling detection of iTRAQ reporter ions. Peptide mass and fragmentation data were used to query an in-house curated version of the IPI database using Mascot (Matrix Science). Protein identifications were validated using a decoy data base. iTRAQ reporter ion-based quantification was performed with in-house developed software. Curve fitting was performed using R software (www.r-project.org).

**iTRAQ labeling of peptides.** For quantitative experiments, reduced and carbamidomethylated kinobead eluates were concentrated on 4-12% NuPAGE gels (Invitrogen) by running sample approximately 1 cm into the gel. After staining with colloidal Coomassie, gels were cut into three slices and subjected to in-gel digestion as described (Rosenfeld et al., 1992, Anal. Biochem. 203, 173-179). Subsequently, peptide extracts were labeled with iTRAQ^{™} reagents (Applied Biosystems) by adding 10 µL reagent in ethanol and incubation for 1 hr at 20°C in 60% ethanol, 40 mM triethylammoniumbicarbonate (TEAB), pH 8.5 (Ross et al., 2004, Mol. Cell. Proteomics 3, 1154-1169). After quenching of the reaction with glycin all labeled extracts of one gel lane were combined and mixed with differently labeled extracts from other competition experiments according to table S9. All experiments were performed in duplicate.

**IMAC enrichment of phospho-peptides.** Samples were subjected to enrichment of phosphorylated peptides by immobilized metal affinity chromatography (IMAC; PhosSelect, Sigma) prior to mass spectrometric analysis as described (Pozuelo et al., 2005, Biochem. J. 392, 163-172).

**LC-MS/MS analysis.** IMAC-binding and non-binding fractions were collected separately, acidified and dried in vacuo. Samples were then resuspended in 0.1 % formic acid in water (non-binding fraction) or 4 mM EDTA, 10 mM TEAB, pH 8.5 in water (phospho-peptide enriched fraction) and injected into a nano-LC system (Eksigent 1D+) which was directly coupled to a LTQ-Orbitrap mass spectrometer (Thermo-Finnigan). Peptides were separated on a custom made 20 cm x 75uM (ID) reversed phase column (Reprosil, Maisch, Germany). Gradient elution was performed from 2% acetonitrile to 40% acetonitrile in 0.1% formic acid within 4 hrs. The LTQ-Orbitrap was operated under the control of XCalibur Developers kit 2.0. Intact peptides were detected in the Orbitrap at 60.000 resolution. Internal calibration was performed using the ion signal from (Si(CH₃)₂O)₆H⁺ at m/z 445.120025 (Olsen et al., 2005, Mol. Cell Proteomics. 4, 2010-2021). Data dependent tandem mass spectra were generated for up to six peptide precursors in the linear ion trap using pulsed-Q dissociation (PQD) to enable detection of iTRAQ reporter ions. For PQD, the Q-value was set to 0.55, activation time was set to 0.32 ms and collision energy of 26 was used. Up to 1E5 ions were accumulated in the ion trap within a maximum ion accumulation time of 1 sec and two spectra were averaged per peptide precursor.

**Peptide and protein identification.** Mascot^{™} 2.0 (Matrix Science) was used for protein identification using 5 ppm mass tolerance for peptide precursors and 0.8 Da tolerance for fragment ions. Carbamidomethylation of cysteine residues and iTRAQ modification of lysine residues were set as fixed modifications and S,T,Y phosphorylation, methionine oxidation, N-terminal acetylation of proteins and iTRAQ modification of peptide N-termini were set as variable modifications. The search data base consisted of an in-house curated version of the IPI protein sequence database combined with a decoy version of this database (Elias et al., 2005, Nat. Methods 2, 667-675). The decoy data base was created using a script supplied by Matrix Science. The Mascot ion score threshold for this database was 38 (indicating <5% random spectrum to sequence assignments). Unless stated otherwise, we accepted protein identifications as follows: i) For single spectrum to sequence assignments, we required this assignment to be the best match *and* a minimum Mascot score of 37 *and* a 10x difference of this assignment over the next best assignment. Based on these criteria, the decoy search results indicate <1% false positive identification rate; ii) For multiple spectrum to sequence assignments and using the same parameters, the decoy search results indicate <0.1% false positive identification rate; iii) for phospho-peptides <5% false positive identification rate was achieved either by a decoy analysis at a minimum Mascot score of 31 or by requiring the identification of a phospho-peptide in at least 12 of the 24 IMAC experiments.

**Peptide and protein quantification.** iTRAQ reporter ion intensities were extracted from raw MS data files using in-house developed software. Only peptides unique for identified proteins were used for relative protein quantification. Fold changes are reported based on iTRAQ reporter ion intensities in comparison to vehicle control and were calculated using a linear model. For quantification of phosphorylated peptides, iTRAQ reporter ion intensities were multiplied with the ion accumulation time yielding an area value proportional to the number of reporter ions present in the ion trap. If multiple spectrum to phospho-peptide assignments were observed within an LC-MS/MS run, these areas were summed-up prior to normalization to vehicle controls. Only phospho-peptides for which the sum of areas was greater than 100.000 were considered for quantitation.

**IC50 calculation.** Dose-response curves were fitted using R (www.r-project.org) and the drc package (www.bioassay.dk). For each protein, relative displacement values to the vehicle control were fitted to concentrations of compound using a 4-parameter, unconstrained log-logistic equation. In some cases, the upper limit had to be fixed to 1 (vehicle control) to allow proper fitting. Inflection point and IC₅₀ (corresponding the 50% of the vehicle control) were reported for any protein that was displaced at least 40% compared to the vehicle control.

### Example 2: Kinase activity assays

Kinase assays were performed using the Invitrogen SelectScreen service. DDR1 activation was assayed in K562 cells as described (L'hote et al., 2002, FASEB J. 16, 234-236). K562 cells were treated with 30 µM pervanadate for 30 minutes to induce tyrosine autophosphorylation of DDR1. DDR1 was analyzed by immunoprecipitation with bead-coupled anti-DDR1 antibodies (Santa cruz sc-532 AC) and Western blotting with anti-DDR1 and anti-phosphotyrosine antibodies. Pre-incubation of the cells with 1 µM imatinib for three hours reduced the pervanadate-induced tyrosine phosphorylation and phosphorylation-mediated degradation of DDR1.

## Claims

1. Use of a Imatinib for the preparation of a medicament for treating a patient having a DDR1 positive cancer.

2. The use of claim 1, wherein the cancer is selected from the group consisting of brain cancer, breast cancer, and lung cancer.

3. The use of any of claims 1 to 2, wherein at least one cancer cell of the patient is tested for the expression of DDR1 prior to the administration of Imatinib and the patient is only treated if DDR1 is expressed in said cancer cell.

4. The use of claim 3, wherein said testing is performed by measuring the presence of DDR1 or by measuring the presence of an mRNA encoding DDR1.

5. The use of claim 4, wherein the presence of DDR1 is measured by immunohistochemistry, Western blot or flow cytometry.

6. The use of claim 4, wherein the presence of the mRNA is measured by quantitative RT-PCR.

7. The use of any of claims 1 to 6, wherein Imatinib is administered in combination with radiation therapy.

8. A method for treating a patient having a DDR1 positive cancer, comprising administering to the patient a pharmacological effective amount of Imantibib.

9. The method of claim 8, wherein the DDR1 positive cancer is selected from the group consisting of brain cancer, breast cancer, and lung cancer.

10. The method of any of claims 8 to 9, wherein at least one cancer cell of the patient is tested for the expression of DDR1 prior to the administration of Imatinib and the patient is only treated if DDR1 is expressed in said cancer cell.

11. The method of claim 10, wherein said testing is performed by measuring the protein level of DDR1 or by measuring the presence of an mRNA encoding DDR1.

12. The method of claim 11, wherein the presence of DDR1 is measured by immunohistochemistry, Western blot or flow cytometry.

13. The method of claim 11, wherein the presence of the mRNA is measured by quantitative RT PCR.

14. The method of any of claims 8 to 13, wherein the Imatinib is administered in combination with radiation therapy.
